# EUROPÄISCHE PATENTANMELDUNG

(11) **EP 1 515 259 A1**
(43) Veröffentlichungstag der Anmeldung: **16.03.2005**
(21) Anmeldenummer: 03020686.6
(22) Anmeldetag: 11.09.2003
(51) Int. Cl.: G06F 19/00

(54) **Verfahren und Programm zur zahnärztlichen Arbeitsvorbereitung**

(71) Anmelder: Pfeiffer, Manfred, Dr., Dubai (AE)
(72) Erfinder: Pfeiffer, Manfred, Dr., Dubai (AE)
(74) Vertreter: Szynka, Dirk, Dr.

(57) **Zusammenfassung**

Die Erfindung betrifft ein Verfahren und ein Computerprogramm sowie ein Datenverarbeitungsgerät zur Arbeitsvorbereitung bei einer zahnärztlichen Behandlung. Dabei werden insbesondere technische Parameter zur Reihenfolge einzelner Behandlungsschritte berücksichtigt.

## Beschreibung

Zahnärzte führen bei der Behandlung des Gebisses von Patienten komplexe technische Verfahren aus, die neben der eigentlichen zahnärztlichen Untersuchung, Behandlung und Kontrolle teilweise auch andere technische Berufsgruppen, etwa Zahntechniker und andere Spezialisten für die Herstellung individuell angepasster technischer Produkte und Laborarbeiten, beinhalten. Diese Verfahren und die zugehörige Arbeitsvorbereitung werden immer anspruchsvoller. Auch werden Datenverarbeitungsgeräte und Computerprogramme zunehmend in Zahnarztpraxen eingesetzt, um die zu bestimmten Behandlungskomplexen gehörenden Daten zu erfassen und zu verwalten.

Im Folgenden wird die zahnärztliche Tätigkeit im Hinblick auf ihre Eigenschaft als technische Tätigkeit betrachtet. Zwar ist die Behandlung des menschlichen Körpers selbst von der Patentierung mangels gewerblicher Anwendbarkeit ausgeschlossen, jedoch ist sie ohne Zweifel technisch.

Gleiches gilt für die zugehörige Arbeitsvorbereitung, also die Ablaufplanung einschl. Projektskizzierung, Konstruktion, Prototypenherstellung etc., die wie bei der Herstellung komplexer technischer Erzeugnisse ebenfalls einen Teil technischer Herstellungsverfahren bildet.

Der vorliegenden Erfindung liegt das technische Problem zugrunde, verbesserte Möglichkeiten zur technischen Arbeitsvorbereitung bei der zahnärztlichen Tätigkeit zur Verfügung zu stellen.

Die Erfindung richtet sich auf ein Verfahren zur zahnärztlichen Arbeitsvorbereitung mit den Schritten:
Aufnahme von Daten zu einem zahnärztlichen Behandlungsprojekt in ein Datenverarbeitungsgerät,
Abrufen von vorbestimmten Ablauftabellendaten aus einem Ablauftabellenspeicher des Datenverarbeitungsgeräts, welche Ablauftabellendaten den Behandlungsprojektdaten zugeordnet sind und zeitliche Parameter zu zu dem Behandlungsprojekt gehörenden Behandlungsschritten enthalten, welche zeitlichen Parameter die zeitliche Reihenfolge von Behandlungsschritten in dem Behandlungsprojekt wiedergeben,
Erstellen eines Behandlungsablaufplans aus den Ablauftabellendaten, welcher eine Mehrzahl Behandlungseinheiten mit jeweiligen Terminen enthält, wobei den Behandlungseinheiten jeweilige Behandlungsschritte zugeordnet sind und die Zuordnung der Behandlungsschritte zu den Behandlungseinheiten und den Terminen die in den Ablauftabellendaten enthaltenen zeitlichen Parameter berücksichtigt,
sowie auf ein entsprechendes Computerprogrammprodukt und auf ein entsprechend programmiertes Datenverarbeitungsgerät.

In der folgenden Beschreibung wird zwischen diesen verschiedenen Patentkategorien nicht mehr im Einzelnen unterschieden, so dass die Offenbarung im Hinblick auf das Verfahren, das Computerprogrammprodukt und auch das Datenverarbeitungsgerät zu verstehen ist.

Die Erfindung geht davon aus, dass bei der zahnärztlichen Arbeitsvorbereitung zunächst Daten zu einem zukünftigen Behandlungsprojekt anfallen, etwa weil der Zahnarzt bei der Untersuchung eines Patienten festgestellt hat, dass ein bestimmter Zahn mit einer Brücke und benachbarte Zähne mit einer Krone zu versehen sind. Der Begriff Behandlungsprojekt meint dabei in einem gemeinsamen Planungszusammenhang geplante und abgewickelte Behandlungsschritte.

Das erfindungsgemäße Verfahren sieht vor, dass die zu dem Behandlungsprojekt gehörenden Daten, die also beispielsweise eine Brücke und zwei Kronen an benachbarten Zähnen kennzeichnen, zum Aufsuchen und Abrufen von zugeordneten Ablauftabellendaten aus einem Ablauftabellenspeicher in einem Datenverarbeitungsgerät genutzt werden. In diesen Ablauftabellendaten sind die technischen Einzelheiten der zu dem Behandlungsprojekt gehörenden Behandlungsschritte abgelegt, wobei zur Veranschaulichung auf das Ausführungsbeispiel verwiesen wird. Insbesondere enthalten die Ablauftabellendaten als wesentlichen Unterschied zu konventionellen Zahnarzt-Verwaltungsprogrammen zeitliche Parameter zu den Behandlungsschritten.

Konventionelle Programme haben sich nämlich darauf beschränkt, die für eine einheitliche und korrekte Rechnungserstellung erforderlichen Informationen zusammenzustellen und ggf. selbsttätig einen Heil- und Kostenplan oder eine Rechnung zu erzeugen. Im Unterschied dazu soll die Erfindung auch in die technische Arbeitsvorbereitung des Zahnarztes eingebunden sein. Dazu enthalten die Ablauftabellendaten technische Details der Behandlungsschritte im Sinne zeitlicher Parameter, und zwar zumindest Parameter, die die zeitliche Reihenfolge von Behandlungsschritten innerhalb des Behandlungsprojekts wiedergeben.

Damit ist es in dem erfindungsgemäßen Verfahren möglich, dem Zahnarzt bei der Arbeitsvorbereitung bereits programmseitig eine vorbereitete Übersicht über die anfallenden Behandlungsschritte in der richtigen zeitliche Reihenfolge und u. U. auch, soweit zutreffend, in der richtigen zeitlichen Zusammengehörigkeit vorzugeben. Der Zahnarzt kann also in ein vorgefertigtes Schema von zeitlich geordneten Behandlungsschritten eingreifen und jeweilige Behandlungssitzungen mit jeweiligen Terminen festlegen. Er ist bei seiner Arbeitsvorbereitung nicht nur dadurch unterstützt, dass die Gefahr gemindert wird, wichtige oder zumindest optional sinnvolle Behandlungsschritte zu vergessen (bei entsprechend anspruchsvoll gepflegten Ablauftabellendaten kann hier auch eine echte Vollständigkeit sichergestellt werden) - es sind zudem technische, d. h. hier die medizinische und die technische Arbeitsvorbereitung betreffende zeitliche Zusammenhänge im Sinne von zeitlichen Reihenfolgen und Gleichzeitigkeiten berücksichtigt und damit entsprechende Fehler ausgeschlossen.

Mit diesen Ablauftabellendaten kann das erfindungsgemäße Verfahren bzw. Programm dann einen Behandlungsablaufplan erstellen, der eine Mehrzahl Behandlungseinheiten mit jeweiligen Terminen enthält. Diese Behandlungseinheiten können Behandlungssitzungen sein, zu denen der Patient die Praxis aufsucht. Es kann sich auch um bestimmte zeitlich festgelegte Behandlungsschritte oder Gruppen von in zeitlichem Zusammenhang auszuführende Behandlungsschritten handeln, die ohne den Patienten in der Praxis, im zuarbeitenden Labor etc. auszuführen sind. Dieser Behandlungsablaufplan berücksichtigt dabei in der Zuordnung der Behandlungsschritte zu den Behandlungseinheiten die genannten zeitlichen Parameter. Die eigentlichen Termine können dabei je nach Ausführungsform vom Zahnarzt oder Praxispersonal, ggf. nach Rücksprache mit den Patienten oder dem Labor, eingetragen oder auch teilweise vom Programm selbsttätig erzeugt werden, etwa wenn bestimmte nicht mit dem Patienten direkt abzustimmende zeitliche Schemata vorab feststehen.

Verkürzt und vereinfacht ausgedrückt sieht die Erfindung also vor, zeitliche Zusammenhänge und die zeitliche Dimension der Arbeitsvorbereitung als technisch relevante Größe in ein Zahnarztprogramm einzuführen und dieses Programm damit für die Arbeitsvorbereitung nutzbar zu machen. Dies ist von besonderer Bedeutung, weil der technische Fortschritt in der Zahnheilkunde zunehmend komplexere Abläufe mit sich bringt und die zeitlichen Zusammenhänge nicht ohne weiteres leicht und fehlerfrei zu überschauen sind. Darüber hinaus muss die Zusammenarbeit verschiedener behandelnder Spezialisten, neben dem Zahnarzt beispielsweise auch der Kieferchirurg, eine spezielle Assistenz oder der Dentaltechniker, und die Beschaffung und Bereitstellung von Material und Hilfsmitteln koordiniert werden.

Vor allem geht die Tendenz zu "Generalsanierungen" des Gebisses, also umfassenden und mehr oder weniger vollständigen Behandlungsprojekten, die im Erfolgsfall lediglich eine Erhaltung des dadurch gewonnen Zustands nach sich ziehen. Andererseits wechselwirken verschiedene Behandlungsschritte am Patienten und insbesondere auch Behandlungsschritte unter Einbeziehung der außerhalb der eigentlichen Arbeit am Patienten selbst stattfindenden Teile der Behandlung stark miteinander, bedingen sich gegenseitig, behindern sich gegenseitig oder können in effizienter Weise gemeinsam durchgeführt werden.

Der Begriff "Behandlungsschritt" meint hier übrigens jeden einzelnen mit der zahnärztlichen Behandlung in Zusammenhang stehenden Teil des Behandlungsprojekts, also auch Zuarbeiten des Labors, Arbeiten des Kieferchirurgen und Anderes, was nicht zur Arbeit des Zahnarztes am Patienten selbst gehört. Der Begriff "Behandlungsschritte" umfasst insbesondere auch reine Untersuchungen und Kontrollen, bei denen also der Patientenkörper oder für diesen vorgesehene Produkte nicht im eigentlichen Sinn manipuliert werden.

Insbesondere sind zwischen verschiedenen Behandlungsschritten häufig relevante Mindestzeitabstände zu beachten, etwa technisch bedingte Mindestvorbereitungsund Arbeitszeiten eines Labors oder Technikers, Mindesteinheilzeiten bis zur Belastbarkeit, Mindestausheilzeiten entzündlicher Prozesse nach einer Behandlung und dgl. Bei einer bevorzugten Ausführungsform sieht die Erfindung vor, dass die zeitlichen Parameter in den Ablauftabellendaten zumindest wesentliche der Mindestzeitabstände zwischen Behandlungsschritten beinhalten und das Programm konsequenterweise reagiert, wenn diese Mindestzeitabstände bei der zeitlichen Planung des Behandlungsprojekts, also bei der Terminzuordnung zu den Behandlungseinheiten, nicht beachtet werden.

Das Programm kann bei dieser Reaktion eine Warnanzeige in Form eines Ausdrucks, einer Bildschirmanzeige oder eines Tonsignals geben oder auch eine (durch den Benutzer aufhebbare) Sperrung der Variation durchführen.

Es ist bevorzugt, dass der Behandlungsablaufplan durch einen Benutzer, etwa den Zahnarzt oder sein Praxispersonal, interaktiv variiert werden kann. Ein Beispiel ist die manuelle Termineingabe. Allerdings umfasst die Erfindung auch Ausführungsformen, bei denen die Terminvergabe ggf. in Abstimmung mit anderen Organisationsprogrammen selbsttätig erfolgt und jedenfalls Änderungen, die die genannten zeitlichen Parameter nicht einhalten würden, nicht vorgenommen werden können.

Insbesondere ist bevorzugt, dass der Benutzer den Behandlungsablaufplan im Laufe der Ausführung des Behandlungsprojekts abhängig von Untersuchungs- und Behandlungsergebnissen variiert und ihn das Programm vorzugsweise auch zur Bestätigung bzw. Änderung einzelner Teile des Behandlungsprojekts abhängig von solchen Ergebnissen auffordert.

Ferner ist bevorzugt, dass für einzelne Behandlungseinheiten, insbesondere Behandlungssitzungen mit dem Patienten, Checklisten erstellt und ausgegeben werden, insbesondere auf einem Papierbogen ausgedruckt oder einem Bildschirm angezeigt werden. Dem Zahnarzt oder Praxispersonal, der/das u. U. eine unmittelbar computergestützte Arbeit nicht gewohnt ist, kann damit in der vertrauten Weise anhand von listenartigen Vorgaben einzelne Behandlungseinheiten durchführen, ohne sich mit dem Behandlungsprojekt insgesamt in dem Datenverarbeitungsgerät befassen zu müssen.

In ähnlicher Weise ist bevorzugt, Terminmerkblätter insbesondere für den Patienten zu erstellen und auszugeben, um eine Einhaltung der vergebenen Termine in konventioneller Form zu gewährleisten.

Ferner können das erfindungsgemäße Verfahren und Programm die Behandlungsprojektdaten neben den bereits geschilderten Aspekten der Arbeitsvorbereitung auch zahnärztlichen Gebührenordnungsziffern oder Ziffern von Laborpreislisten, wie etwa der GOÄ, der GOZ, der BEB/BEL, zuordnen und damit eine Einbeziehung der buchhalterischen Aspekte des Behandlungsprojekts oder eine Verzahnung mit einer diesbezüglichen Verwaltung erleichtern. Insbesondere kann das Programm selbsttätig Rechnungsvorschläge einschl. Laborrechnungsvorschlägen und/oder Kostenpläne erstellen.

Die Erfindung stellt sich zum einen als technisches Arbeitsverfahren dar. Des weiteren bezieht sich die Erfindung natürlich auf ein dieses Verfahren ermöglichendes Computerprogramm in in ein Datenverarbeitungsgerät geladener Form, auf einen Datenträger gespeicherter Form oder irgendeiner anderen Form. Schlussendlich bezieht sich die Erfindung auch auf ein mit dem erfindungsgemäßen Computerprogramm versehenes Datenverarbeitungsgerät, das dementsprechend an das erfindungsgemäße Verfahren angepasst ist. Der Begriff "Datenverarbeitungsgerät" umfasst dabei nicht nur Einzelgeräte sondern auch komplexe Datenverarbeitungsanlagen, Rechnernetze und dgl.

Die Erfindung wird im Übrigen anhand des folgenden Ausführungsbeispiels näher erläutert, wobei die dabei offenbarten Einzelmerkmale auch in anderen Kombinationen erfindungswesentlich sind und die Beschreibung natürlich nur beispielhaft erfolgt, also nicht den Gegenstand der Erfindung einschränkt.

Im Einzelnen zeigt:
- Fig. 1: eine schematische Übersicht über den Verfahrensablauf bei der zahnärztlichen Behandlung eines Patienten nach konventioneller Vorgehensweise;
- Fig. 2: einen beispielhaften Verfahrensablauf eines Behandlungsprojekts zur Verdeutlichung von Zeitparametern der Behandlungsschritte;
- Fig. 3: einen zweiten beispielhaften Verfahrensablauf eines Behandlungsprojekts;
- Fig. 4: einen dritten beispielhaften Verfahrensablauf eines Behandlungsprojekts;
- Fig. 5: ein schematisches Diagramm zur Verdeutlichung der Arbeitsweise des erfindungsgemäßen Programms;
- Fig. 6: einen beispielhaften Bildschirminhalt des in Fig. 5 dargestellten Programms;
- Fig. 7: eine analog Fig. 1 aufgebaute Darstellung des Verfahrensablaufs gemäß der Erfindung.

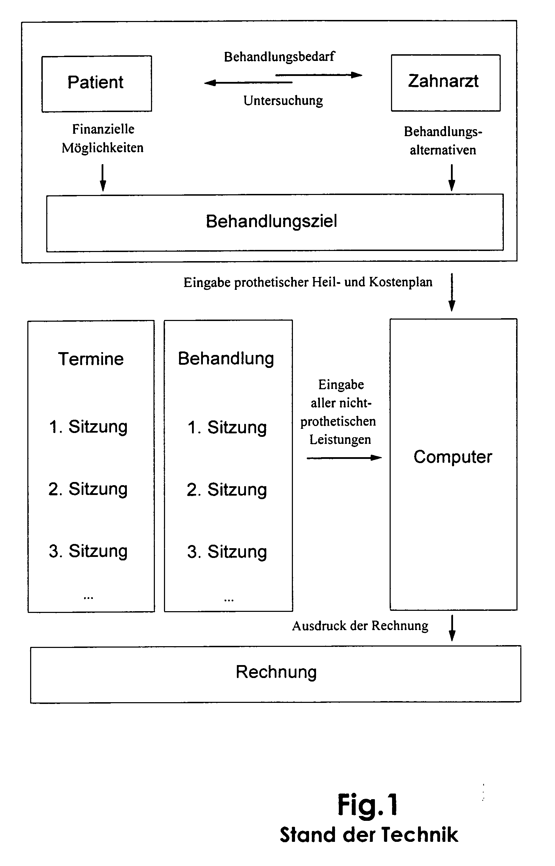

Üblicherweise erfolgt die Benutzung eines Zahnarzt-Computerprogramms in Zusammenhang mit der zahnärztlichen Behandlung bislang wie in Fig. 1 schematisch dargestellt. Der Patient vermittelt dem Zahnarzt seine Beschwerden und seine Vorgeschichte und soweit für ihn erkennbar seinen Behandlungsbedarf. Der Zahnarzt führt eine erste Untersuchung durch, auf deren Basis in Abstimmung mit den finanziellen Möglichkeiten des Patienten zwischen verschiedenen Behandlungsalternativen ausgewählt wird und ein Behandlungsziel definiert wird. Dieses Behandlungsziel ist die dem Patienten vermittelte Zusammenfassung eines Behandlungsprojekts, das im Folgenden ablaufen soll.

Das Behandlungsprojekt gliedert sich in vielen Fällen in eine Vorbehandlung, eine prothetische Versorgung sowie eine Nachkontrolle und evtl. Korrekturen. Für den prothetischen Teil wird ein Heil- und Kostenplan erstellt, der den Hauptanteil der Gesamtkosten abdeckt. Dies erfolgt über ein Computerprogramm, dem auch während dem Ablauf des Behandlungsprojekts über verschiedene Behandlungseinheiten weitere Leistungen mitgeteilt werden. Die Behandlungseinheiten sind hier eine erste, eine zweite und eine dritte Sitzung mit verschiedenen Terminen.

Die Erstellung des Zeitplans für die Terminierung der Behandlung erfolgt unabhängig von dem Computerprogramm. Das Computerprogramm dient vielmehr zur Sammlung der Behandlungsdaten zwecks Erstellung von Heil- und Kostenplänen und zwecks Erleichterung der Rechnungserstellung, evtl. vollautomatischer Rechnungserstellung.

Wesentlich an diesem Stand der Technik ist, dass die zeitliche Planung der einzelnen Sitzungen unabhängig vom Heil- und Kostenplan erfolgt und das Computerprogramm die erbrachten Leistungen nur aus buchhalterischen Gründen sammelt und nur buchhalterisch zwischen diesen Leistungen differenzieren kann. Das Computerprogramm unterstützt also nicht bei der Arbeitsvorbereitung sondern lediglich bei der Buchhaltung und Abrechnung.

Andererseits bestehen zunehmend komplexe Zusammenhänge zwischen einzelnen Behandlungsschritten in Behandlungsprojekten des Zahnarztes. Zum Ersten ist dies durch den technischen Fortschritt in der Zahnheilkunde bedingt, zum Zweiten sind zunehmend verschiedene Personen innerhalb der Zahnarztpraxis und auch außerhalb der Zahnarztpraxis in ihrer Zusammenarbeit zu koordinieren und zum Dritten besteht ein Trend zu den bereits erwähnten "Generalsanierungen".

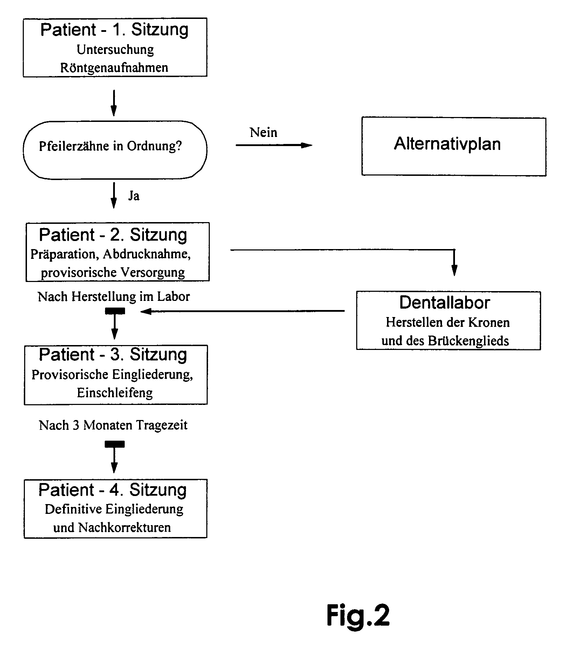

Der Übersichtlichkeit halber sollen hier keine besonders komplexen Beispiele dargestellt werden. Stattdessen zeigt Fig. 2 ein vereinfachtes Schema für ein einfacheres Behandlungsprojekt, nämlich die Eingliederung einer Brücke im Unterkiefer.

In einer ersten Behandlungssitzung wird der Patient untersucht und werden evtl. bereits Röntgenaufnahmen vorgenommen. Nach diesen kann entschieden werden, ob die als Pfeiler für die Brücke dienenden Zähne dafür geeignet sind und im negativen Fall ein Alternativplan aufgestellt werden, der hier nicht näher ausgeführt wird. Im positiven Fall wird mit dem Patienten eine zweite Sitzung vereinbart, bei der eine Präparation, eine Abdruckentnahme sowie eine provisorische Versorgung erfolgt.

Der Abdruck wird einem Dentallabor zur Verfügung gestellt, das dementsprechend Kronen und das Brückenglied herstellt. Diese Herstellung im Labor erfordert eine gewisse technische Bearbeitungszeit. Beispielsweise könnte hier eine Bearbeitungszeit von acht Arbeitstagen in Betracht kommen. Mit einem dementsprechenden zeitlichen Mindestabstand ist dann eine dritte Behandlungssitzung mit dem Patienten vereinbart worden, in der eine provisorische Eingliederung und ein Einschleifen erfolgt. Der Patient muss nun eine gewisse Tragezeit hinter sich bringen. Beispielsweise kann eine Zeit von drei Monaten angebracht sein, um einer evtl. traumatisierten Pulpa die notwendige Ausheilung zu ermöglichen. Die dreimonatige Tragezeit gibt also einen weiteren zeitlichen Mindestabstand zwischen der dritten Behandlungssitzung und einer darauf folgenden vierten Behandlungssitzung vor, in der eine definitive Eingliederung und evtl. Nachkorrekturen erfolgen.

Im vorliegenden einfachen Beispiel kommen als Zeitparameter also die technische Bearbeitungszeit des Dentallabors als Mindestabstand zwischen zweiter Behandlungssitzung und dritter Behandlungssitzung sowie die dreimonatige Ausheilzeit als Mindestzeitabstand zwischen dritter Behandlungssitzung und vierter Behandlungssitzung und zudem die technisch bedingte Reihenfolge dieser Behandlungsteile in Betracht.

Andererseits sollten provisorische Versorgungen in Abhängigkeit von der Fertigungsqualität nicht übermäßig lange im Mund des Patienten verbleiben, weil sonst deren weiterer Halt nicht gewährleistet ist und die Gefahr einer Kariesbildung entstehen kann. Daher ist ein maximaler Zeitabstand zwischen der zweiten Behandlungssitzung und der dritten Behandlungssitzung von vier Wochen als Beispielswert sinnvoll. Auch wenn dieser Maximalzeitabstand in vielen Fällen nicht zum Tragen kommen wird, weil ohnehin nach Patientenwunsch in möglichst rascher Folge Termine vergeben werden, so kommt es doch gerade bei berufstätigen Patienten immer wieder zu Absagen oder auch zu erst nachträglich erkannten Terminüberschneidungen in der Praxis. Werden dann neue Termine vereinbart, so ist es von erheblichem technischen Vorteil, zu gewährleisten, dass der Zeitabstand zwischen der zweiten Behandlungssitzung und der dritten Behandlungssitzung nicht übermäßig groß wird.

Analoge Argumente gelten für den Zeitabstand zwischen der dritten Behandlungssitzung und der vierten Behandlungssitzung. Hier sollte beispielsweise eine Tragezeit des nur provisorisch eingegliederten Kronen-/Brückensystems von einem Monat nicht überschritten werden.

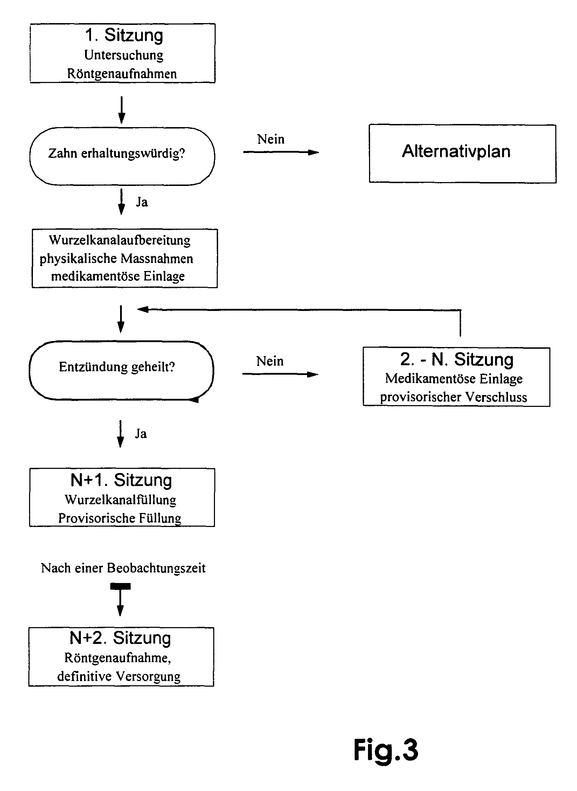

Ein weiteres Beispiel zur Veranschaulichung zeitlicher Parameter zeigt Fig. 3. Hier geht es um die konservierende Wurzelbehandlung bei einer grangänösen Pulpa. Die zerstörte Pulpa muss entfernt werden und der Wurzelkanal danach aufbereitet und gefüllt werden.

In einer ersten Behandlungssitzung erfolgt wieder eine Untersuchung und nach Möglichkeit bereits die Bewertung von Röntgenaufnahmen. Wie im vorherigen Beispiel soll auf einen im negativen Fall aufzustellenden Alternativplan nicht eingegangen werden. Im positiven Fall kommt es zur Wurzelkanalaufbereitung, zu hier nicht näher auszuführenden physikalischen Maßnahmen und einer medikamentösen Einlage, mit der die Entzündung beseitigt werden soll.

Nach einem gewissen zeitlichen Mindestabstand von drei Tagen wird nun untersucht, ob die akute Entzündung abgeheilt ist. Dieser zeitliche Mindestabstand ist technisch sinnvoll, weil eine vorzeitige Untersuchung mit nur sehr geringer Wahrscheinlichkeit zu einem positiven Ergebnis führen wird. Andererseits ist auch ein maximaler zeitlicher Abstand zu berücksichtigen, weil der aufbereitete Wurzelkanal noch nicht gefüllt ist. Hier kommen etwa sechs Monate in Betracht. Bei der ersten Behandlungssitzung zur Überprüfung wird nun entschieden, ob die Entzündung ausgeheilt ist. Im negativen Fall kommt es zu einer zweiten oder auch weiteren Sitzung zur Wiederüberprüfung, möglicherweise mit weiterer medikamentöser Einlage und provisorischem Verschluss. Nach dieser ersten Überprüfungssitzung und nach weiteren Sitzungen können wiederum zeitliche Mindestabstände und Maximalabstände beachtet werden von beispielsweise drei Tage bzw. sechs Monaten.

Wenn die Entzündung ausgeheilt ist, kann in derselben Behandlungssitzung oder in einer neu zu vereinbarenden Behandlungssitzung dann die Wurzelkanalfüllung erfolgen. Nach einer Beobachtungszeit von mindestens sechs bis neun Monaten erfolgt eine weitere Untersuchung, insbesondere eine Röntgenaufnahme und im positiven Fall eine Versorgung der Zahnkrone.

Dieses Beispiel verdeutlicht bereits eine größere Zahl von zeitlichen Parametern im Sinne von minimalen und maximalen Zeitabständen zwischen einzelnen Behandlungsschritten bzw. einzelnen Behandlungseinheiten. Es versteht sich, dass keine verbindlichen Aussagen darüber getroffen werden können, in welchen Fällen Mindestzeitabstände und in welchen Fällen Maximalzeitabstände zu beachten sind und wann nicht. Dies ist zum einen eine Frage der Sorgfalt des behandelnden Zahnarztes und zum anderen auch eine Frage der interindividuellen Variabilität der Patienten und der im Einzelnen verwendeten Materialien.

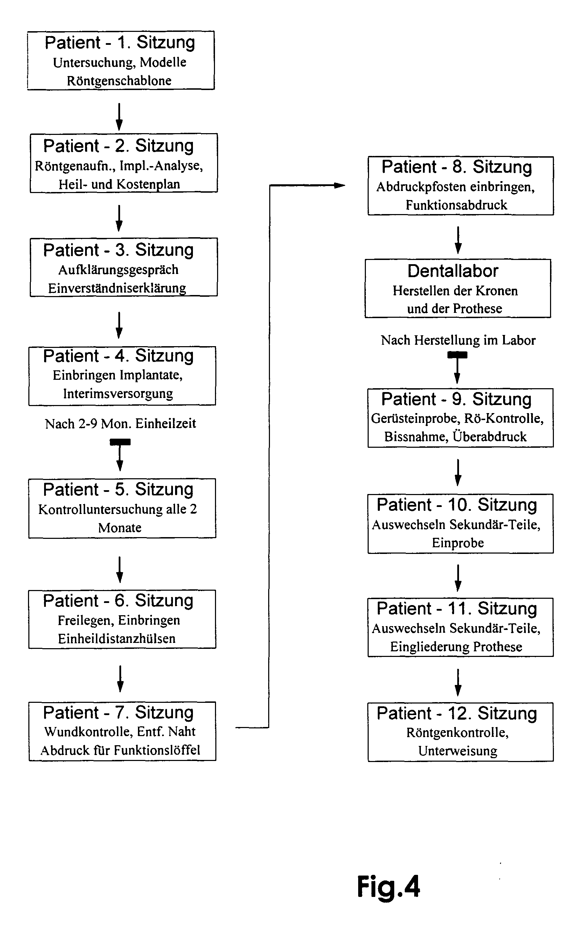

Ein drittes Beispiel in Fig. 4 zeigt schematisch den Behandlungsablauf bei einer sog. Cover-Denture-Prothese im Unterkiefer, die auf Implantaten verankert wird.

In einer ersten Behandlungssitzung erfolgt eine Untersuchung und die Erstellung von Modellen zur Fertigung einer Röntgenschablone. In einer zweiten Behandlungssitzung erfolgen Röntgenaufnahmen, eine Implantatanalyse sowie die Erstellung eines Heil- und Kostenplans. In einer dritten Behandlungssitzung kommt es dann zu einem detaillierten Aufklärungsgespräch mit Einverständniserklärung des Patienten, so dass in einer vierten Behandlungssitzung die Implantate eingebracht werden können. Nach einer im Bereich von zwei bis vier Monaten für Unterkiefer und sechs bis neun Monaten für Oberkiefer liegenden Mindesteinheilzeit erfolgt eine Kontrolluntersuchung im Rahmen einer fünften Behandlungssitzung, woraufhin in einer sechsten Behandlungssitzung die Implantate chirurgisch freigelegt und Einheildistanzhülsen eingebracht werden.

In einer siebten Behandlungssitzung erfolgt, wiederum mit einem bestimmten zeitlichen Mindestabstand zur sechsten Behandlungssitzung von einer Woche eine Wundkontrolle, die Entfernung der Naht und ein Abdruck für Funktionslöffel, woraufhin nach einem weiteren zeitlichen Mindestabstand von zwei Wochen in einer achten Behandlungssitzung Abdruckpfosten eingebracht werden können und ein Funktionsabdruck erfolgen kann. Daraufhin stellt das Dentallabor in einer typischen Bearbeitungszeit von drei Wochen die Kronen und Prothesen her, so dass es mit einem entsprechenden zeitlichen Mindestabstand zu einer neunten Behandlungssitzung mit dem Patienten kommt, die eine Gerüsteinprobe, eine Röntgenkontrolle, eine Bissnahme und einen Überabdruck beinhaltet.

In einer zehnten Behandlungssitzung werden Sekundärteile ausgewechselt, und es erfolgt eine Einprobe, in einer elften Behandlungssitzung werden wiederum Sekundärteile ausgewechselt, und es wird die Prothese eingegliedert, woraufhin nach einem weiteren Zeitabstand in einer zwölften Behandlungssitzung eine Röntgenkontrolle mit Patientenunterweisung erfolgt.

Die oben dargestellte Behandlung erstreckt sich über mindestens acht Monate, wobei nicht nur die Arbeiten des Zahnarztes, sondern auch die eines Kieferchirurgen und des Dentallabors sowie die Material- und Hilfsmittelbeschaffung zeitlich und fachlich zu koordinieren sind. Sie veranschaulicht den durch eine computergestützte Arbeitsvorbereitung und -koordinierung erzielbaren Qualitäts- und Sicherheitsgewinn. Andererseits sollte der Behandlungszeitraum nicht über achtzehn Monate im Oberkiefer und neun Monate im Unterkiefer hinausgehen, weil die Trabekelstruktur des Knochens sich erst unter Belastung bildet. Diese Maximalzeit betrifft genaugenommen den Abstand zwischen vierter und elfter Sitzung.

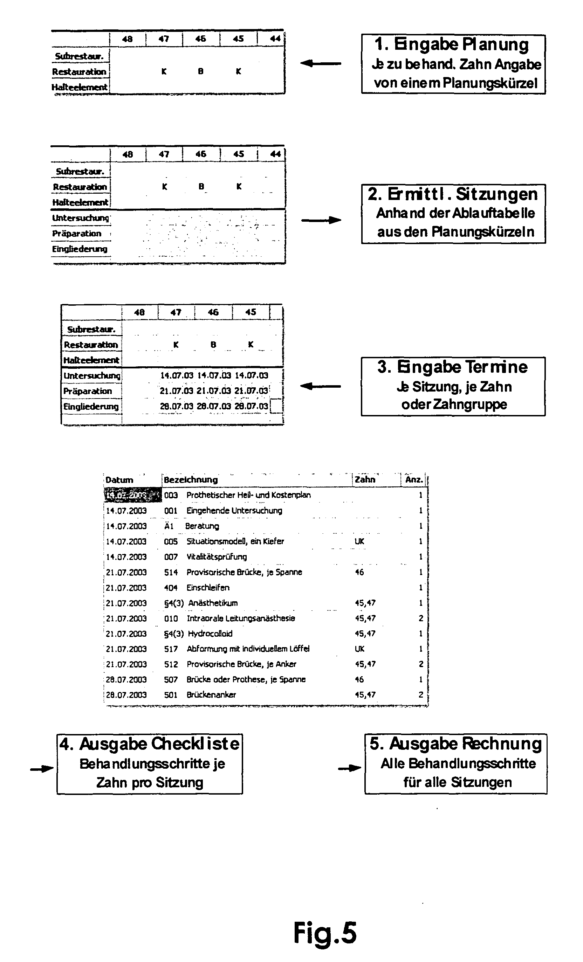

Fig. 5 veranschaulicht nun, wie hierbei das erfindungsgemäße Computerprogramm genutzt werden kann. Zunächst werden in einem in Fig. 5 oben links ausschnittsweise dargestellten Matrixschema sog. Planungskürzel für jeden zu behandelnden Zahn eingegeben. Dies entspricht der bisher so bezeichneten Aufnahme von Daten zu dem Behandlungsprojekt. Beispielsweise werden hier für den (aus Sicht des Patienten) unteren rechten Kieferteil die Zähne 5 - 7, also nach üblicher Nummerierung die Nummern 45 - 47, im Sinne einer Restauration eine Krone, ein Brückenglied und eine weitere Krone hierfür als Planungskürzel K, B, K eingegeben. Die Planungskürzel als aufgenommene Daten dienen dem Programm dazu, in vorbestimmten Ablauftabellen eines Ablauftabellenspeichers entsprechende Ablauftabellendaten zu den Planungskürzeln abzurufen. Beispielsweise werden für die Kronen und das Brückenglied jeweils Felder für Behandlungssitzungen zur Untersuchung, zur Präparation und zur Eingliederung geöffnet, wie das Diagramm zeigt. Das Programm kennt anhand der Ablauftabellendaten aber überdies die einzelnen Behandlungsschritte zu diesen Behandlungsteilen.

In einem nächsten Schritt kann der Zahnarzt einer ersten Behandlungssitzung einen Termin zuweisen, hier den 14.07.2003 für eine Untersuchung in Bezug auf alle drei Zähne 45 - 47. Im weiteren Verlauf kann entweder das Programm zu den folgenden Terminen für die Präparation und die Eingliederung, also zu den folgenden beiden Behandlungsteilen, Terminfenster vorgeben, die vorgegebene Mindest- und Maximalzeitabstände berücksichtigen, oder frühest mögliche Termine angeben, die vom Zahnarzt nach Absprache mit dem Patienten verschoben werden können, oder auch nur eine frei vom Zahnarzt eingegebene Terminwahl auf die Einhaltung zeitlicher Parameter prüfen. Beispielsweise kann dies hier so erfolgen, dass nach Eingabe des 14.07.2003 für den zur Untersuchung gehörenden Behandlungsteil das Computerprogramm den zweiten Termin für die Präparation zur freien Eingabe vorgibt. Hier hat der Zahnarzt nun den 21.07.2003 eingegeben. Daraufhin kann das Programm unter Beachtung einer Mindestbearbeitungszeit von einer Woche für die Herstellung der Kronen und Brücke den 28.07.2003 als Eingliederungstermin vorschlagen, der in diesem Fall vom Zahnarzt und Patienten akzeptiert wurde. Bein einer Verschiebung hätte das Programm einen maximalen Zeitabstand zwischen Präparationstermin und Eingliederungstermin von beispielsweise vier Wochen berücksichtigt.

Das untere große Feld in Fig. 5 zeigt nun beispielhaft verschiedene Behandlungsschritte, die den verschiedenen Behandlungsteilen, also Sitzungen, zugeordnet sind. Diese Behandlungsschritte sind mit vor dem Klartext stehenden Kurzbezeichnungen bereits entsprechenden Kostenziffern oder Kostenvorschriften zugeordnet und werden in den beiden letzten Spalten bestimmten Zähnen und Kieferteilen zugewiesen und in ihrer Anzahl quantifiziert. Dieses Schema kann für die jeweiligen Behandlungssitzungen als Checkliste für den Zahnarzt und auch für das Praxispersonal dienen. Es erlaubt jedoch bereits davor eine technisch-inhaltlich qualifizierte und insbesondere zeitlich aufgelöste Übersicht über das gesamte Behandlungsprojekt im Sinne eines Arbeitsvorbereitungsplans. Im Übrigen kann das Computerprogramm natürlich auch so ausgestaltet sein, dass es mit Hilfe der vorliegenden Daten zu den entsprechenden Zeitpunkten bei der Rechnungserstellung hilft, beispielsweise am Ende des gesamten Behandlungsprojekts eine Gesamtrechnung für alle Sitzungen erstellt.

Das erfindungsgemäße Programm beschränkt sich also nicht auf eine buchhalterische Unterstützung sondern erlaubt eine hinsichtlich der Berücksichtigung zeitlicher Parameter technisch qualifizierte Arbeitsvorbereitung, insbesondere Terminplanung, Material- und Hilfsmittelbeschaffung, und die Erstellung von Checklisten und damit eine verbesserte Kontrolle der Arbeitsdurchführung und der Beschaffung.

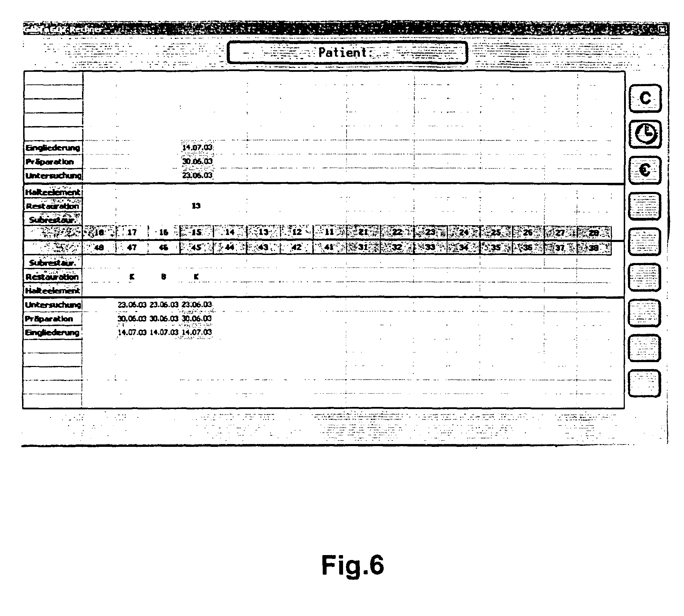

Fig. 6 zeigt zur Veranschaulichung einen Gesamtbildschirminhalt bei der Terminfestlegung entsprechend Fig. 5, wobei hier beide Kiefer des Patienten in Gänze dargestellt sind. In Fig. 6 sind von Fig. 5 abweichende Termine eingetragen und zusätzlich für den Zahn 15 im linken Oberkiefer für einen mit dem Planungskürzel 13 symbolisierten Teil des Behandlungsprojekts Maßnahmen für die drei Behandlungstermine am 23.06, 30.06. und 14.07.2003 festgelegt. Dieser Teil des Behandlungsprojekts soll nicht im Einzelnen dargestellt werden. Fig. 6 verdeutlicht, dass das Programm in sehr übersichtlicher Weise mit einer vertikalen Zeitachse und einer horizontalen Zahnzuordnung und den dem Zahnarzt und Praxispersonal vertrauten Planungskürzeln einen matrixartigen Überblick über das gesamte Behandlungsprojekt bietet.

Die im rechten Bereich mit C, dem Uhrensymbol und dem Eurosymbol beschriebenen Schaltflächen dienen zum Löschen von Eingaben, zur Terminfestlegung bzw. zum Umschalten in ein Buchhaltungsmodul zur Rechnungserstellung oder Erstellung eines Heil- und Kostenplans.

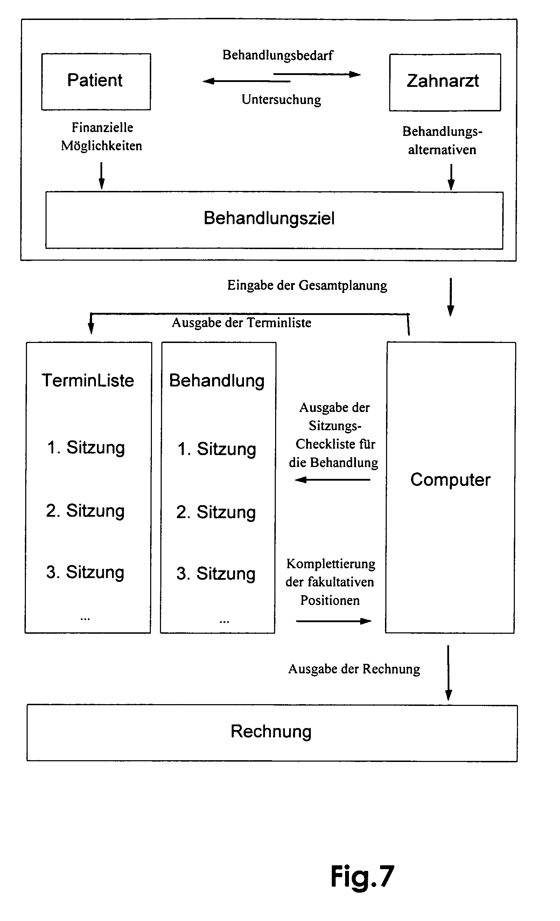

Fig. 7 dient zum Vergleich mit Fig. 1 und zeigt den Ablauf des Behandlungsprojekts in Übersichtsdarstellung. Der obere Kasten entspricht dabei Fig. 1 und muss daher nicht neu erläutert werden. Zu ergänzen bleibt allenfalls, dass dem Zahnarzt die Beratung des Patienten hinsichtlich seines Behandlungsbedarfs, der damit verbundenen Kosten und der zur Verfügung stehenden Alternativen durch die Übersichtsfunktion des Computerprogramms erleichtert wird. Es können also auch versuchsweise Behandlungsprojekte geplant werden, um die Beratung zu stützen und zu veranschaulichen. Nicht gewünschte Behandlungsprojekte können dann wieder gelöscht werden. In der beschriebenen Weise wird die eigentlich vorgesehene Behandlung als Behandlungsprojekt geplant, wie dies bereits anhand der Fig. 5 und 6 dargestellt wurde. Dabei können neben der Übersicht über die Gesamtplanung Terminlisten und Sitzungschecklisten für die einzelnen Behandlungssitzungen und auch andere Behandlungsteile, wie Laborarbeit, ausgegeben werden. Während des Ablaufs des Behandlungsprojekts können im Übrigen Änderungen und Rückmeldung über das Vorliegen oder Weglassen fakultativer Positionen erfolgen. Während des gesamten Behandlungsprojekts haben alle beteiligten Personen überdies die Möglichkeit sich über die Planung und Arbeitsvorbereitung zu informieren und ihre Arbeitsschritte dementsprechend einzurichten und zu überprüfen.

Am Ende des Behandlungsprojekts kann wiederum, wie im Stand der Technik auch, eine Gesamtabrechnung erfolgen. Allerdings kann hier die Rechnungserstellung zusätzlich eine zeitliche Differenzierung, also eine Zuordnung einzelner Rechnungsposten zu bestimmten Behandlungssitzungen vornehmen. Damit wird im Sinne einer Kostentransparenz und Glaubwürdigkeitssteigerung dem Patienten die Zuordnung der Kosten zu der erfolgten Behandlung besser nachvollziehbar.

## Patentansprüche

1. Verfahren zur zahnärztlichen Arbeitsvorbereitung mit den Schritten:
- Aufnahme von Daten zu einem zahnärztlichen Behandlungsprojekt in ein Datenverarbeitungsgerät,
- Abrufen von vorbestimmten Ablauftabellendaten aus einem Ablauftabellenspeicher des Datenverarbeitungsgeräts, welche Ablauftabellendaten den Behandlungsprojektdaten zugeordnet sind und zeitliche Parameter zu zu dem Behandlungsprojekt gehörenden Behandlungsschritten enthalten, welche zeitlichen Parameter die zeitliche Reihenfolge von Behandlungsschritten in dem Behandlungsprojekt wiedergeben,
- Erstellen eines Behandlungsablaufplans aus den Ablauftabellendaten, welcher eine Mehrzahl Behandlungseinheiten mit jeweiligen Terminen enthält, wobei den Behandlungseinheiten jeweilige Behandlungsschritte zugeordnet sind und die Zuordnung der Behandlungsschritte zu den Behandlungseinheiten und den Terminen die in den Ablauftabellendaten enthaltenen zeitlichen Parameter berücksichtigt.

2. Verfahren nach Anspruch 1, bei dem die zeitlichen Parameter Mindestzeitabstände zwischen Behandlungsschritten wiedergeben.

3. Verfahren nach Anspruch 1, bei de die zeitlichen Parameter Maximalzeitabstände zwischen Behandlungsschritten wiedergeben.

4. Verfahren nach einem der vorstehenden Ansprüche, bei dem in den Behandlungsprojektdaten Daten zu nichtzahnärztlichen Tätigkeiten, etwa Labor, Herstellung von Vorrichtungen oder Beschaffung von Materialien und/oder Hilfsmitteln, enthalten sind.

5. Verfahren nach einem der vorstehenden Ansprüche, bei dem der Behandlungsablaufplan durch einen Benutzer interaktiv variiert wird.

6. Verfahren nach Anspruch 5, bei dem die Variationen des Behandlungsablaufplans hinsichtlich der Einhaltung der in den Ablauftabellendaten enthaltenen zeitlichen Parameter kontrolliert werden und im Fall von Nichteinhaltung zumindest eine Sperrung der Variation oder eine Warnanzeige des Datenverarbeitungsgeräts erfolgt.

7. Verfahren nach Anspruch 6, bei dem der Behandlungsablaufplan im Laufe der Ausführung des Behandlungsprojekts ansprechend auf dabei auftretende Untersuchungs-/Behandlungsergebnisse variiert wird.

8. Verfahren nach einem der vorstehenden Ansprüche, bei dem Behandlungssitzungschecklisten erstellt und ausgegeben werden

9. Verfahren nach einem der vorstehenden Ansprüche, bei dem Terminmerkblätter erstellt und ausgegeben werden.

10. Verfahren nach einem der vorstehenden Ansprüche, bei dem die Behandlungspojektdaten zahnärztlichen Gebührenordnungsziffern zugeordnet werden.

11. Verfahren nach Anspruch 10, bei dem das Datenverarbeitungsgerät selbsttätig Rechnungsvorschläge einschl. Laborrechnungsvorschläge erstellt.

12. Verfahren nach Anspruch 11, bei dem das Datenverarbeitungsgerät selbsttätig Kostenpläne erstellt.

13. Computerprogrammprodukt, das so ausgelegt ist, dass nach Laden des Computerprogramms in ein Datenverarbeitungsgerät dieses an die Durchführung des Verfahrens nach einem der vorstehenden Ansprüche angepasst ist.

14. Datenverarbeitungsgerät, in dem ein Computerprogramm gemäß Anspruch 13 geladen ist und das demzufolge an die Durchführung des Verfahrens nach einem der Ansprüche 1 - 12 angepasst ist.

## Geänderte Patentansprüche

### Geänderte Patentansprüche gemäss Regel 86(2) EPÜ.

**1.** Die Recherchenabteilung des Europäischen Patentamts hat nach Regel 45 EPÜ eine Erklärung abgegeben, die für das weitere Verfahren für die Anmeldung als europäischer Recherchenbericht dient. Diese Erklärung hindert jedoch den Prüfer nich daran, zu gegebener Zeit während des Verfahrens eine Recherche zum Stand der Technik durchzuführen, soweit er dies für zweckmäßig hält. Gegenwärtig erachtet es der Prüfer als nicht notwendig.

**2.** Die gegenwärtige Anmeldung betrifft ein administratives bzw. organisatorisches Problem, nämlich die Verbesserung der zahnärztlichen Arbeitsvorbereitung mit Hilfe eines Datenverarbeitungsgeräts. Das Verfahren (nach Anspruch 1) umfaßt drei wesentliche Schritte: Aufnahme von Daten [...], Abrufen von vorbestimmten Ablauftabellendaten [...], sowie Erstellen eines Behandlungsablaufplans [...].
2.1 Der Prüfer stellt nicht in Frage, daß die Implementierung des Verfahrens nach Anspruch 1 der Anmeldung technische Merkmale beinhaltet, da es in der Tat ein Datenverarbeitungsgerät erfordert, welches mit einem Datenverarbeitungsprogramm für die Verwaltung ausgestattet ist. Diese technischen Merkmale sind jedoch auf eine Art und Weise miteinander verknüpft, die kein technisches Problem löst.
2.2 Der Prüfer ist der Auffassung, daß die vorliegende Anmeldung die Lösung des gestellten Problems auf einer sehr hohen, abstrakten Ebene - der aministrativen Ebene - löst. Nur abstrakte Merkmale werden in der Anmeldung offenbart. Das interne Wirken dieser Merkmale wird ebenfalls auf einer hohen Abstraktionsebene beschrieben. Es werden keinerlei Details über die Funktionsweise des die Methode implementiertenden Programms offenbart. Die Anmeldung als Ganzes gibt keinen Hinweis auf ein zu lösendes technisches Problem bei der Implementierung der beschriebenen Methode (nach Anspruch 1).

**3.** Der Anmelder wird im übrigen darauf hingewiesen, daß der Prüfer die Verwendung allgemeiner technischer Merkmale wie ein Datenverarbeitungsgerät und ein Datenverarbeitungsprogramm in dem Verfahren der vorliegenden Anmeldung nicht als erfinderisch im Sinne des Artikel 56 EPÜ ansieht, so daß das Erfordernis des Artikels 52(1) EPÜ nicht erfüllt ist. Der Prüfer stellt fest, daß der Gegenstand der Ansprüche der Anmeldung mangels erfinderischer Tätigkeit nicht patentierbar ist, da keine technische Lösung eines erkennbaren technischen Problems offenbart wird und keine technischen Effekte oder Ergebnisse, die für den Fachmann unerwartet oder überraschend wären, ersichtlich sind. Vielmehr stellt das angegebene Verfahren zur zahnärztlichen Arbeitsvorbereitung eine bloße Automatisierung von bisher vom Menschen ausgeführten Funktionen dar, die dem allgemeinen Trend in der Technik entgegenkommt und daher nicht als erfinderisch angesehen werden kann (Entscheidung der Beschwerdekammer T775/90).
